# EUROPÄISCHE PATENTSCHRIFT

(11) **EP 3 164 101 B1**
(45) Veröffentlichungstag und Bekanntmachung des Hinweises auf die Patenterteilung: **13.03.2024**
(21) Anmeldenummer: 15745145.1
(22) Anmeldetag: 03.07.2015
(51) Int. Cl.: A61F 5/37, A61B 6/04, A61F 13/00, A61G 13/10, A61G 1/044, A61G 13/12, A61B 90/14, A61B 46/23

(54) **VORRICHTUNG ZUR FIXIERUNG**
DEVICE FOR IMMOBILIZATION
DISPOSITIF D'IMMOBILISATION

(30) Priorität: 03.07.2014 DE 202014005396 U
(43) Veröffentlichungstag der Anmeldung: 10.05.2017
(73) Patentinhaber: Isys Medizintechnik GmbH, 6370 Kitzbühel (AT)
(72) Erfinder: VOGELE, Michael, 86830 Schwabmünchen (DE)
(74) Vertreter: Keller Schneider Patentanwaltsgesellschaft mbH
(86) Internationale Anmeldenummer: PCT/EP2015/001352
(87) Internationale Veröffentlichungsnummer: WO 2016/000825

(56) Entgegenhaltungen:
- EP-A2- 2 191 801
- WO-A1-94/13177
- WO-A1-99/11452
- WO-A1-99/33408
- DE-A1- 19 640 366
- DE-A1- 19 937 066
- DE-A1-102012 100 559
- DE-A1-102012 211 358
- DE-U1-202011 005 573
- US-A- 3 982 132
- US-A- 5 800 346
- US-A1- 2007 235 038

## Beschreibung

Die Erfindung betrifft eine Vorrichtung zur Fixierung nach den oberbegrifflichen Merkmalen des Anspruches 1. Unter "Fixierung" sollen hierbei im medizinischen Sinne insbesondere die Immobilisierung, Ruhigstellung, Komprimierung und/oder Positionierung des menschlichen Körpers bzw. Teile des menschlichen Körpers (Beine, Arme) verstanden werden, wobei insbesondere auch medizinische Zielvorrichtungen, Marker und/oder chirurgische Instrumenten für bildgestützte, minimalinvasive Operationsverfahren zuverlässig am Patienten fixiert werden sollen.

In vielen Bereichen der Humanmedizin oder der medizinischen Forschung ist eine sichere Fixierung/Immobilisierung/Ruhigstellung bzw. Positionierung des Patienten oder Körperteile des Patienten oder die (mechanische) Anbringung von Vorrichtungen oder Geräten erforderlich. Von größter Bedeutung ist dies insbesondere auf dem Gebiet der diagnostischen und therapeutischen Radiologie, der Strahlentherapie oder bei operativen/chirurgischen Eingriffen (z. B. Neurochirurgie), aber auch bei der prä- oder postoperativen Versorgung.

Durch die Einbeziehung der Computertechnologie in Diagnose und Therapie sind die Anforderungen an Genauigkeit und Reproduzierbarkeit sowohl bei der Festlegung eines stereotaktischen Rahmensystems am Menschen als auch bei der Fixierung des Körpers selbst gestiegen. Komfort, Schnelligkeit in der Anwendung, Mobilität und Kosten spielen dabei eine erhebliche Rolle, wobei eine invasive Fixation (durch Verschraubungen etc.) ohnehin möglichst vermieden werden sollte.

Bei diesen sog. non-invasiven Fixationsarten sind als Stand der Technik bekannt:
a) Fixation des Körpers mit Bändern oder Manschetten:
   Der Körper des Patienten liegt hierbei auf einer Schaumstoffunterlage und quer über den Körper gespannte Bänder fixieren den Patienten auf diese Unterlage. Nachteilig ist hierbei, dass es durch starken Zug der Bänder zu Druckstellen, Verschiebungen und/oder Hautschwellungen kommen kann (inhomogene Druckverteilung insbesondere an den Rändern der gespannten Bänder oder Gurte).
b) Fixation durch Schalungen:
   Hierbei wird der Patient auf eine Art "Luftmatratze" gelegt, welche mit Schaumstoffkügelchen gefüllt ist. Durch Absaugen der Luft in dieser Matratze verfestigt sich diese durch Aneinanderlegen der Schaumstoffkügelchen. Dabei wird die Vakuummatratze im ersten Schritt zunächst angepasst und im zweiten Schritt dann weiter abgesaugt. Nachteilig ist hierbei, dass die üblicherweise verwendeten "Matratzen" zwar eine Ruhigstellung, aber keine exakte Fixation garantieren. Bei unkooperativen Patienten ist eine ausreichende Ruhigstellung praktisch nicht möglich. Zudem können durch Faltenbildung oder zu starkes Andrücken ebenfalls oft Druckstellen entstehen, welche vor allem bei narkotisierten Patienten zu Gewebeverletzungen führen können.

### a) Vakuumfixationssystem:

Bei Vakuumfixationssystemen wird Vakuum an den Körper angelegt. Durch Absaugen der Luft an der Patientenoberfläche kann eine gute Fixierung geschaffen werden. Nachteilig ist hierbei, dass die Vakuumpumpe permanent laufen muss. Zudem ist das Vakuumsystem relativ komplex, daher schlecht in der Handhabung und schlecht transportabel. Bei Eingriffen, die hohe Keimfreiheit oder sogar Sterilität erfordern, stellt der Luftstrom der Vakuumpumpe ein Risiko dar (Keimverschleppung). Höhere Fixierkräfte sind jedoch kaum möglich, weil durch lang anhaltende Drücke Verletzungen (z. B. Minderdurchblutung, Blutergusse etc.) entstehen können. Bei einem Ausfall des Vakuums geht die Fixierung schlagartig verloren, so dass eine Gefahr für den Patienten entstehen kann oder der (chirurgische/radiologische) Eingriff abgebrochen bzw. wiederholt werden muss.

Andere Techniken wie Schienen, Kunststoffabdruck, Gipse, etc. weisen ähnliche Nachteile auf. Zusätzlich sind diese Methoden noch mit erheblichem finanziellen bzw. zeitlichen Aufwand verbunden und werden deshalb nurfür Langzeitanwendungen verwendet.

Beispielsweise ist aus der DE 10 2012 100 559 A1 ein Patientenfixiersystem bekannt, das eine Mehrzahl von Fixierblöcken umfasst, welche jeweils zumindest eine unterseitige Oberfläche, die zumindest in einem von äußeren Kräften Zustand eine geometrische Bezugsebene aufspannt, zumindest eine oberseitige Oberfläche und eine oder mehrere seitliche Oberflächen aufweisen. An der oberseitigen Oberfläche ist vollflächig oder teilflächig zumindest ein Klettverschluss-Hakenfeld oder Klettverschluss-Flauschfeld zur lösbaren Befestigung von Fixierelementen wie bspw. von Gurten vorgesehen.

Eine weitere Fixierungsvorrichtung, insbesondere eine Retraktorfixierung, ist aus der US 5 800 346 A bekannt. Dort umfasst die Fixierungsvorrichtung ein Band aus biegsamem Material mit gegenüberliegenden Befestigungsendbereichen, an denen sich Hakenelemente oder ähnliches befinden, um in gegenüberliegende Seiten eines Operationstisches einzugreifen. Entlang des Bands sind Taschen mit offenen Enden zur Aufnahme des proximalen Endes eines Retraktorgriffs vorgesehen. Die Hakenelemente können beispielsweise mittels Schlaufen befestigt werden, die mittels Klettverschlüssen ausgebildet sind.

Weiterhin ist eine Fixierung, insbesondere eine Baby-Rückhaltevorrichtung, aus der WO 94/13177 A1 bekannt. Die Baby-Rückhaltevorrichtung dient zur Verwendung mit einer Baby-Wickelauflage. Die Baby-Rückhaltevorrichtung umfasst Basiselemente mit Befestigungsmitteln zur Befestigung an der Oberseite einer Wickelauflage an einer ersten Stelle und an ihrer Oberseite ausgebildete Eingriffsmittel zum lösbaren Eingriff in mindestens zwei Rückhalteelemente. Die Eingriffsmittel können als Klettverschlüsse ausgebildet sein.

Der Erfindung liegt deshalb die Aufgabe zugrunde, eine Vorrichtung zur Fixierung zu schaffen, welche die genannten Nachteile vermeidet, einfach im Aufbau sowie in der Anwendung in hohem Maße patientenschonend ist. Die Vorrichtung soll darüber hinaus die exakte Anbringung von Eichpunkten (sog. Markern) und/oder Zielvorrichtungen und/oder sonstigem medizinischen Zubehör (wie z.B. Instrumenten-/Endoskophalter, MR-Spule(n), etc.) ermöglichen und bei Bedarf eine optimale Zugänglichkeit zu Operationsgebieten ermöglichen.

Diese Aufgabe wird durch eine Vorrichtung mit den Merkmalen des Anspruches 1 gelöst. Vorteilhafte Ausgestaltungen sind Gegenstand der Unteransprüche.

Ein wesentliches Merkmal ist hierbei ein verfestigter Vliesstoff, mit dem ein stabiles Fixieren am menschlichen Körper ermöglicht wird, da sich die Vliesfasern mit dem Mikroklettverschluss verschlingen. Der z.B. durch Recken oder unter Vorstreckung gesteuertes Aufwickeln auf eine Rolle verfestigte Vliesstoff weist dabei eine definierte Zugfestigkeit auf und dient selbst als Gegenelement für einen Mikroklettverschluss. Die ansonsten übliche doppelte Ausführung eines Klettverschluss mit Hakenelementen und Gegenelementen wird somit vermieden, da sich der Vliesstoff sicher verhakt. Die Randbereiche der Vliesstreifen sind jedoch im Gegensatz zu Gurten sehr nachgiebig, so dass Hautschädigungen des Patienten ausgeschlossen werden können. Der je nach Körperregion mehr oder weniger (quer-)elastische Vliesstoff passt sich optimal der Kontur des Patienten an, womit eine optimale Druckverteilung garantiert ist und Druckstellen vermieden werden. Somit kann der Patientenkörper in Relation zu einer Grundplatte eines Untersuchungs- oder Operationstisches fixiert werden, wobei durch leichten Druck eine leichte, unschädliche Kompression des Körpers erreicht wird. Als Nebeneffekt kann die Kompression auch noch vor, während und nach der Behandlung genutzt werden, indem man z.B. eine Wundkompresse zwischen Vlies und Wunde/Eingriffsregion einlegt und dadurch eine Wundkompression erzeugt. Im Gegensatz zu Gurten oder Bändern mit aufgenähtem Klettverschluss werden somit nicht mehr zwei zusammenwirkende Klettelemente benötigt; vielmehr wirkt der verfestigte Vliesstoff als ein Verbindungselement, in das sich die Haken oder Pilze des Mikroklettverschlusses (wieder lösbar) einkrallen. Somit sind die Kosten für eine derartige Vorrichtung zur Fixierung des menschlichen Körpers bzw. von Körperteilen sehr gering, da praktisch nur der handelsüblich preiswert erhältliche Vliesstoff einen Verbrauchsartikel bildet. Dieser ist dabei in Art einer Mullbinde einfach anwendbar, wobei auch die Hygieneanforderungen eingehalten werden. Bei Bedarf (z.B. direktem Kontakt mit der Wunde/dem Operationsgebiet) kann das Vlies auch steril eingesetzt werden.

Der verwendete Mikroklettverschluss ist bevorzugt als flexible Folie mit Pilzen und/oder Haken als Verbindungselemente zum Vliesstoff hin ausgeführt. Dabei kann diese Folie z.B. als Streifen mit seiner von den Haken abgewandten Seite an der Seitenfläche eines OP-Tisches aufgeklebt werden, um so als stabile Verankerung zu dienen. Solch ein Mikroklettverschluss ist bevorzugt aus Polypropylen (PP) oder Polyamid (PA) hergestellt, um auf einfache Weise gereinigt zu werden. Die Haken/Pilze der Mikroklettfolie sind relativ klein (Submillimeterbereich), so dass bei Anwendung von Reinigungslösungen ein Kapillareffekt entsteht und damit eine vollständige Desinfektion des Mikroklettverschlusses. Klassische Klett- oder Hakenbänder haben diese Eigenschaft nicht; sie sind deshalb sehr schlecht zu reinigen bzw. zu desinfizieren und in Krankenhäusern - vor allem im OP oder Sterilbereich mit hohen Hygieneanforderungen - nur sehr bedingt einsetzbar. Das andere Element des Fixierelements, nämlich der Vliesstoff ist meist als (steriler) Einwegartikel ausgebildet, so dass dieser nach Hautkontakt ähnlich einer Mullbinde einfach zu entsorgen ist-

Der Vliesstoff ist meist streifenartig ausgebildet und kann zur Schaffung einer Zugangsöffnung als Eingriffsstelle zu einem Körperteil (z.B. Punktionsöffnung) Durchbrechungen oder regelmäßige Ausstanzungen (z. B. von 1 cm Durchmesser mit z. B. je 10 cm Abstand in Längsrichtung) aufweisen. Damit kann der Vliesstoff auf die vorgesehene Eingriffsstelle gelegt werden und dann beidseitig verspannt werden. Alternativ kann der Vliesstoff eine längliche Aussparung aufweisen, deren Länge länger ist als die den Körper überspannenden Teils des Vliesstoffs und deren Breite genügend ist, um an einem gewünschten, überspannten Körperteil einen Eingriff durchzuführen. Dabei ist die zur Befestigung verfügbare Fläche des Vliesstoffs nicht durch die Aussparung verringert, wodurch die Halteeigenschaften am Mikroklettverschluss nicht vermindert werden. Hierbei ist der Vliesstoff bevorzugt von einer Rolle abwickelbar sowie bevorzugt individuell ablängbar (z. B. mit Schere etc.). Es können jedoch auch im Vliesstoff z. B. jeden Meter oder im etwas größeren Abstand als dem zwischen den Mikroklettverschlüssen (etwas länger als Bettbreite) Perforierungen als Soll-Trennstelle vorgesehen sein. Die Vliesbänder können auch überlappen oder in mehreren Lagen übereinander gelegt werden; dabei kann der mechanische Zug jedes einzelnen Vliesstreifens "individuell" eingestellt werden, wodurch unterschiedliche Körperregionen unterschiedlich stark komprimiert und fixiert werden können. Die Verwendung mehrerer, überlappender und/oder übereinander gelegter Vliesstreifen ist auch ein Sicherheitsaspekt, da hierdurch sichergestellt werden kann, dass durch Lösen eines oder mehrerer Bänder nicht die gesamte Fixation des Patienten verloren geht. Neben der streifenartigen Ausführung sind auch tuch- oder westenartige Ausführungen des Vliesstoffs denkbar; der Vorteil ist eine noch schnellere und noch einfachere Handhabung.

Der verfestigte Vliesstoff ist im Allgemeinen in Spann- bzw. Längsrichtung (des Streifens) zugfest und in Querrichtung dazu (Streifenbreite) elastischer ausgebildet, um sich perfekt am Patientenkörper anzuschmiegen. Die Vliesverfestigung kann dabei mit verschiedenen Methoden erfolgen, wie dies in WIKIPEDIA unter "Vliesstoffe" ausführlich erläutert wird, insbesondere als sog. Nadelvliese. Wesentlich ist jedoch, dass der Vliesstoff atmungsaktiv und damit patientenschonend bleibt. Zudem können im Vliesstoff sog. Marker und/oder Verstärkungen integriert sein.

Wie erwähnt, kann der Mikroklettverschluss an den Randbereichen eines Untersuchungs- oder OP-Tisches befestigt sein, insbesondere in Profilierungen entlang den Kanten verschiebbar oder am Tisch angeklebt sein. Es können hierbei die Standard-Profilierungen verwendet werden, wie man diese bei OP-Tischen, Patientenliegen, CT- oder MRT-Tischen etc. vorfindet. Daneben kann der Mikroklettverschluss in Form von Patches mit Klebestreifen versehen sein sowie auch einfach auf Lagerungszubehör (Kopfschale, Vakuumkissen etc.) aufgeklebt oder anderweitig angebracht werden. Auf dem Fixierelement kann zudem eine Zusatzeinrichtung, insbesondere Marker- oder Zielvorrichtung befestigt werden, bevorzugt mittels wenigstens einer Adapterplatte, die ebenfalls Klett-Verschlusselemente tragen kann oder auch eine Klebeschicht. Somit kann auch die Außen- bzw. Oberseite des Vliesstoffes zur Verankerung genutzt werden.

Nach erfolgter Fixierung und steriler Abdeckung kann z. B. ein Eingriff (ggf. mit Roboterunterstützung) in einem OP-Fenster stattfinden. Dabei können die o. g. Marker für bildgebende Verfahren oder die OP-Navigation reproduzierbar angebracht werden. Da es sich durch den Vliesstoff um ein großflächiges, ergonomisches Fixierelement handelt, entstehen hohe Haltekräfte bei maximalem Komfort. Die Haftkräfte können noch gesteigert werden, wenn auf den Vliesstoff im Kontaktbereich zum Patienten hin ein hautverträglicher Sprühkleber oder ein dünnes, doppelseitiges Klebeband aufgebracht wird. Hierdurch können auch Weichteilgewebe fixiert werden, um diese z. B. aus dem Bestrahlungsfeld fernzuhalten.

Ausführungsbeispiele werden anhand der Zeichnung beschrieben. Hierbei zeigen:
Fig. 1 das Anbringen einer Vorrichtung zur Fixierung;
Fig. 2 eine vergrößerte Detailansicht der Vorrichtung; und
Fig. 3 das Aufsetzen/Anbringen zusätzlicher Vorrichtungen.

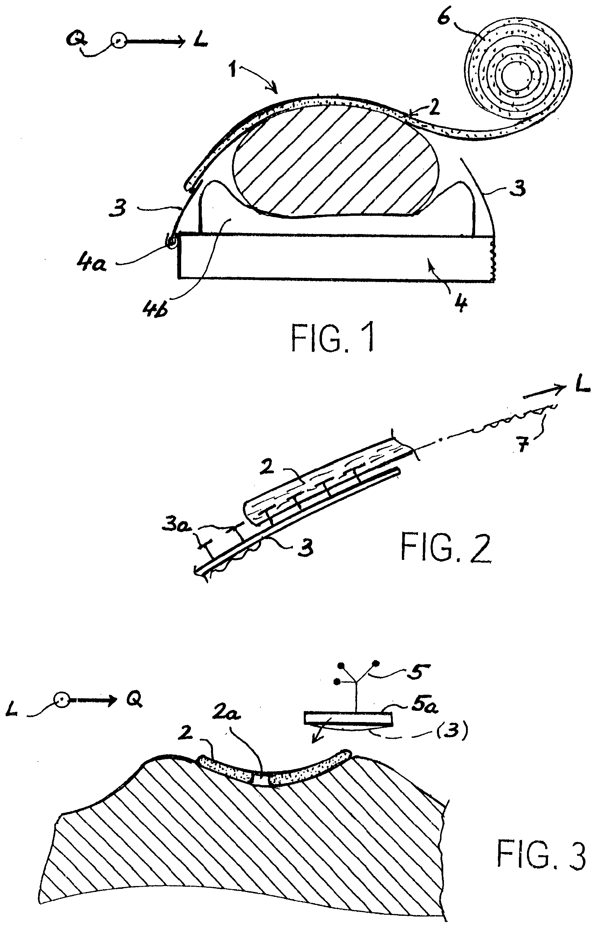

Die Vorrichtung zur Fixierung des menschlichen Körpers bzw. von Körperteilen besteht aus wenigstens einem Fixierelement 1, das auf der Körperoberfläche (schraffiert dargestellt; hier z. B. ein Querschnitt eines Rückens, der auf einer Polsterunterlage 4b aufliegt) positionierbar ist (Fig. 1). Dies erfolgt mit einem verfestigten Vliesstoff 2, der auf die Haut aufgelegt wird, um dann links und rechts der Polsterunterlage 4b an je einem Mikroklettverschluss 3 befestigt zu werden (durch Verkrallung der Haken im Mikrometerbereich; daher die vorstehende Bezeichnung). Der Vliesstoff 2 ist bevorzugt streifenartig ausgebildet und insbesondere auf einer Rolle 6 aufgewickelt. Der Vliesstoff 2 ist in seiner Anbringrichtung L (meist quer zur Körperlängsachse) zugfest und in seiner Querrichtung Q dazu relativ elastisch ausgebildet ist. An der Außenseite des so befestigten Fixierelements 1 kann eine Adapterplatte 5a angebracht sein, um dort Marker 5 (oder sonstige Zusatzeinrichtungen) stabil und reproduzierbar zu verankern (vgl. Fig. 3). Zur Fixierung an einer Grundplatte eines Untersuchungs- oder Operationstisches 4 ist bevorzugt mindestens eine Profilierung 4a, z.B. eine gerundete Nut vorgesehen. Es können auch mehrere Fixierelemente 1 z. B. am Rücken mit Abstand zueinander angeordnet sein. Hierdurch werden dann entsprechende Operationsbereiche freigelassen.

In Fig. 2 ist eine stark vergrößerte Darstellung der Verbindung zwischen Vliesstoff 2 und dem Mikroklettverschluss 3 gezeigt, wobei sich die im Mikrometerbereich kleinen, pilz- oder hakenförmigen Verbindungselemente 3a (hier als T eingezeichnet) mit den Fasern des Vliesstoffes 2 vielfach verkrallen. Der Mikroklettverschluss 3 ist hier bevorzugt an der Unterseite (zum Patienten hin) in Art eines Klebebandes ausgeführt, um am OP-Tisch 4 angeklebt zu werden (vgl. Fig. 1, rechts). Ebenso kann solch ein "Patch" auch an der Seitenfläche der Patientenunterlage 4b festgeklebt werden, je nach Lage des zu fixierenden Körperteils, also z. B. im Schulterbereich und/oder im Hüftbereich und/oder im Beinbereich usw.. Die Fasern des Vliesstoffes 2 verlaufen weitgehend in Längsrichtung L und sorgen somit für die hohe Zugfestigkeit. Auf der hier rechten Seite ist außerhalb des Mikroklettverschlusses 3 (also zum Patienten hin) noch eine Klebstoffschicht 7 angedeutet, die bevorzugt als Sprühkleber kurz vor dem Fixieren aufgebracht wird. Hierdurch lassen sich die Anhaftkräfte weiter steigern oder Weichteilbereiche aus dem OP-Bereich (vgl. Zugangsöffnung 2a in Fig. 3) definiert fernhalten.

Wie in Fig. 3 im Querschnitt gezeigt kann im streifenförmigen Vliesstoff 2 eine Öffnung 2a (z. B. für eine Punktion im Lendenwirbelbereich) vorgesehen ist. Somit kann der behandelnde Arzt oder eine Behandlungsperson praktisch jede Körperregion (ganzer Körper oder nur Körperteile) je nach Bedarf fixieren. Dabei ergibt sich eine universelle Adaption an OP-Tische und eine einfache Fixierung von chirurgischen Geräten und Markern. Zusätzlich denkbar ist eine Unterpolsterung des Vliesstoffes 2 (Erhöhung des Komforts), wobei der Vliesstoff 2 im Gegensatz zu üblichen Bändern oder Manschetten die Hautatmung ermöglicht, also auch bei längerer Anwendung nicht reizend wirkt. Insgesamt werden so Körperteile mit definiertem Druck und sehr geringen Kosten fixiert, wobei sich die vorgeschlagene Vorrichtung auch mit einer auf Adhäsivkräfte beruhenden Vorrichtung des Anmelders (vgl. DE 20 2011 005 573) kombinieren lässt. Das Prinzip des Mikroklettverschlusses 3 im Zusammenwirken mit dem Vliesstoff 2 lässt sich auch zur Befestigung der o.g. Adapterplatte 5a anwenden, wie in Fig. 3 angedeutet ist. Dabei kann die Unterseite der Adapterplatte 5a (ebenfalls mit Mikroklettverschluss versehen) auf der Außenseite des verspannten Vliesstoffes 2 verankert werden. Der Vliesstoff 2 kann auch über die o.g. Adapterplatte 5a hinweg geführt werden, um diese noch zusätzlich zu befestigen. Dies gilt auch für andere medizinische Zusatzvorrichtungen, wie MR-Spulen oder Drucksensoren zur Erfasssung von (Atem-) Bewegungen des Patienten, welche durch den Vliesstoff 2 körpernah (wie eine zweite Haut) sicher fixiert werden.

## Patentansprüche

1. Vorrichtung zur Fixierung des menschlichen Körpers bzw. von I<örperteilen, insbesondere zur Anbringung von medizinischen Zielvorrichtungen, Markern oder chirurgischen Instrumenten für bildgestützte, minimalinvasive Operationen, mit mindestens einem Fixierelement (1), das auf der I<örperoberfläche positionierbar ist,
**dadurch gekennzeichnet, dass**
die Vorrichtung einen Mikroklettverschluss (3) aufweist,
das Fixierelement (1) aus einem verfestigten Vliesstoff (2) gebildet ist, der an dem Mikroklettverschluss (3) befestigbar ist und mittels des Mikroklettverschlusses (3) verspannbar ist,
der Mikroklettverschluss (3) im Mikrometerbereich kleine pilz- oder hakenförmige Verbindungselemente (3a) aufweist, die dazu ausgebildet sind, sich mit Fasern des Vliesstoffes (2) vielfach zu verkrallen, und
der Vliesstoff (2) in Längsrichtung (L) zugfester und in Querrichtung (Q) elastischer ausgebildet ist.

2. Vorrichtung nach Anspruch 1, **dadurch gekennzeichnet, dass** der Mikroklettverschluss (3) als flexible Folie ausgeführt ist.

3. Vorrichtung nach Anspruch 1 oder 2, **dadurch gekennzeichnet, dass** der Mikroklettverschluss (3) aus Polypropylen (PP) oder Polyamid (PA) besteht.

4. Vorrichtung nach einem der Ansprüche 1 bis 3, **dadurch gekennzeichnet, dass** der Vliesstoff (2) streifen- oder tuchartig ausgebildet ist.

5. Vorrichtung nach Anspruch 4, **dadurch gekennzeichnet, dass** der Vliesstoff (2) mindestens einer Zugangsöffnung (2a) zu einem I<örperteil aufweist.

6. Vorrichtung nach einem der Ansprüche 1 bis 5, **dadurch gekennzeichnet, dass** der Vliesstoff (2) von einer Rolle (6) abwickelbar ist.

7. Vorrichtung nach Anspruch 6, **dadurch gekennzeichnet, dass** der Vliesstoff (2) an Perforationen ablängbar ist.

8. Vorrichtung nach einem der Ansprüche 1 bis 7, **dadurch gekennzeichnet, dass** der Vliesstoff (2) als steriler Einwegartikel ausgebildet ist.

9. Vorrichtung nach einem der Ansprüche 1 bis 8, **dadurch gekennzeichnet, dass** im Vliesstoff (2) Marker und/oder Verstärkungen integriert sind.

10. Vorrichtung nach einem der Ansprüche 1 bis 9, **dadurch gekennzeichnet, dass** der Mikroklettverschluss (3) an den Randbereichen eines Untersuchungs- oder OP-Tisches (4) befestigt ist.

11. Vorrichtung nach Anspruch 10, **dadurch gekennzeichnet, dass** der Mikroklettverschluss (3), in Profilierungen (4a) entlang den Kanten verschiebbar ist.

12. Vorrichtung nach Anspruch 10, **dadurch gekennzeichnet, dass** der Mikroklettverschluss (3) am bzw. auf dem Tisch (4) und/oder einer Patientenunterlage (4b) angeklebt ist.

13. Vorrichtung nach einem der Ansprüche 1 bis 12, **dadurch gekennzeichnet, dass** am Fixierelement (1) wenigstens eine Zusatzeinrichtung, insbesondere eine Zielvorrichtung (5) oder Elemente zur Bildgebung fixierbar ist.

14. Vorrichtung nach Anspruch 13, **dadurch gekennzeichnet, dass** die Fixierung mittels wenigstens einer Adapterplatte (5a), die ebenfalls den Mikroklettverschluss (3) trägt.

15. Vorrichtung nach einem der Ansprüche 1 bis 14, **dadurch gekennzeichnet, dass** auf den Vliesstoff (2) eine Klebstoffschicht (7) aufgebracht ist, insbesondere in Form eines hautverträglichen Sprühklebers oder dünnen, doppelseitigen Klebebands.

## Claims

1. A device for immobilization of the human body or of body parts, in particular for attachment of medical target devices, markers or surgical instruments for image-guided, minimally invasive surgery, with at least one fixing element (1) to be positioned on the body surface, **characterized in that**
the device comprises a micro hook-and-loop fastener (3),
the fixing element (1) comprises a consolidated non-woven fabric (2) which can be fastened to the micro hook-and-loop fastener (3) and can be tensioned by the micro hook-and-loop fastener (3),
the micro hook-and-loop fastener (3) comprises small connecting elements (3a) in the micrometer range in the form of mushrooms or hooks, which are configured to interlock with fibres of the nonwoven fabric (2) in multiple positions, and
the non-woven fabric (2) has high tensile strength in the longitudinal direction (L) and is formed resilient in the transverse direction (Q).

2. The device according to claim 1, **characterized in that** the micro hook-and-loop fastener (3) is formed as a flexible film.

3. The device according to claim 1 or 2, **characterized in that** the micro hook-and-loop fastener (3) is made of polypropylene (PP) or polyamide (PA).

4. The device according to any one of claims 1 to 3, **characterized in that** the non-woven fabric (2) is a strip or cloth.

5. The device according claim 4, **characterized in that** the non-woven fabric (2) comprises at least one access opening (2a) to a body part.

6. The device according to any one of claims 1 to 5, **characterized in that** the non-woven fabric (2) can be unwound from a roll (6).

7. The device according to claim 6, **characterized in that** the non-woven fabric (2) can be cut to length at perforations.

8. The device according to any one of claims 1 to 7, **characterized in that** the non-woven fabric (2) is formed as a sterile disposable article.

9. The device according to any one of claims 1 to 8, **characterized in that** markers and/or reinforcements are integrated in the non-woven fabric (2)

10. The device according to any one of claims 1 to 9, **characterized in that** the micro hook-and-loop fastener (3) is fastened at edge regions of an examination or operating table (4),

11. The device according to claim 10, **characterized in that** the micro hook-and-loop fastener (3) is movable in profiles (4a) along the edges.

12. The device according to claim 10, **characterized in that** the micro hook-and-loop fastener (3) is adhered to the table (4) and/or a patient support (4b).

13. The device according to any one of claims 1 to 12, **characterized in that** at least one additional device, in particular a target device (5) or elements for imaging can be fixed to the fixing element (1).

14. The device according to clam 13, **characterized in that** the fixation is achieved by means of at least one adapter plate (5a), which also carries the micro hook-and-loop fastener (3).

15. The device according to any one of claims 1 to 14, **characterized in that** an adhesive layer (7) is applied to the non-woven fleece (2), in particular in the form of a skin-compatible spray adhesive or thin double-sided adhesive tape.

## Revendications

1. Dispositif de fixation du corps humain ou de parties du corps, en particulier pour la mise en place de dispositifs de visée médicaux, de marqueurs ou d'instruments chirurgicaux pour des opérations mini-invasives assistées par imagerie, avec au moins un élément de fixation (1) qui est configuré afin de pouvoir être positionné sur la surface du corps,
**caractérisé en ce que**
le dispositif comporte une microfixation auto-agrippante (3),
l'élément de fixation (1) est formé d'un non-tissé consolidé (2) qui est configuré pour pouvoir être fixé à la microfixation auto-agrippante (3) et serré au moyen de la microfixation auto-agrippante (3),
la microfixation auto-agrippante (3) présente des attaches (3a) en forme de champignons ou de crochets de l'ordre du micromètre, qui sont conçues pour s'accrocher de manière multiple aux fibres du non-tissé (2), et
le non-tissé (2) est plus résistant à la traction dans le sens longitudinal (L) et plus élastique dans le sens transversal (Q).

2. Dispositif selon la revendication 1, **caractérisé en ce que** la microfixation auto-agrippante (3) est réalisée sous forme de film flexible.

3. Dispositif selon la revendication 1 ou 2, **caractérisé en ce que** la microfixation auto-agrippante (3) est en polypropylène (PP) ou en polyamide (PA).

4. Dispositif selon l'une des revendications 1 à 3, **caractérisé en ce que** le non-tissé (2) est en forme de bande ou de tissu.

5. Dispositif selon la revendication 4, **caractérisé en ce que** le non-tissé (2) présente au moins une ouverture d'accès (2a) à une partie du corps.

6. Dispositif selon l'une des revendications 1 à 5, **caractérisé en ce que** le non-tissé (2) est configuré pour pouvoir être déroulé à partir d'une bobine (6).

7. Dispositif selon la revendication 6, **caractérisé en ce que** le non-tissé (2) peut être coupé à la longueur au niveau de perforations.

8. Dispositif selon l'une des revendications 1 à 7, **caractérisé en ce que** le non-tissé (2) est conçu comme un article stérile à usage unique.

9. Dispositif selon l'une des revendications 1 à 8, **caractérisé en ce que** des marqueurs et/ou des renforts sont intégrés dans le non-tissé.

10. Dispositif selon l'une des revendications 1 à 9, **caractérisé en ce que** la microfixation auto-agrippante (3) est fixée sur les bords d'une table d'examen ou d'opération (4).

11. Dispositif selon la revendication 10, **caractérisé en ce que** la microfixation auto-agrippante (3) est déplaçable dans des profilés (4a) le long des bords.

12. Dispositif selon la revendication 10, **caractérisé en ce que** la microfixation auto-agrippante (3) est collée sur la table (4) et/ou sur un support (4b) du patient.

13. Dispositif selon l'une des revendications 1 à 12, **caractérisé en ce qu'**au moins un dispositif supplémentaire, en particulier un dispositif de visée (5) ou des éléments pour l'imagerie, est fixable sur l'élément de fixation (1).

14. Dispositif selon la revendication 13, **caractérisé en ce que** la fixation est réalisée au moyen d'au moins une plaque d'adaptation (5a) qui porte également la microfixation auto-agrippante (3).

15. Dispositif selon l'une des revendications 1 à 14, **caractérisé en ce qu'**une couche de colle (7) est appliquée sur le non-tissé (2), notamment sous la forme d'une colle en spray compatible avec la peau ou d'un ruban adhésif double face mince.
